# FASCICULE DE BREVET EUROPEEN

(11) **EP 2 665 507 B1**
(45) Date de publication et mention de la délivrance du brevet: **11.02.2015**
(21) Numéro de dépôt: 11811078.2
(22) Date de dépôt: 12.12.2011
(51) Int. Cl.: A61M 16/00, A61M 16/08, A61B 5/00

(54) **DISPOSITIF ET PROCÉDÉ DE DÉTECTION D'EAU CONDENSÉE DANS LE PASSAGE DE GAZ D'UNE INSTALLATION DE TRAITEMENT DE L'APNÉE OBSTRUCTIVE DU SOMMEIL**
VORRICHTUNG UND VERFAHREN ZUR ERKENNUNG VON KONDENSIERTEM WASSER IM LUFTWEG EINER BEHANDLUNGSEINHEIT FÜR OBSTRUKTIVE SCHLAFAPNOE
DEVICE AND METHOD FOR DETECTING CONDENSED WATER IN THE GAS PASSAGE OF AN OBSTRUCTIVE SLEEP APNEA TREATMENT UNIT

(30) Priorité: 20.01.2011 FR 1150440
(43) Date de publication de la demande: 27.11.2013
(73) Titulaire: L'Air Liquide Société Anonyme pour l'Etude et l'Exploitation des Procédés Georges Claude, 75007 Paris (FR)
(72) Inventeur: CARLOS, Géraldine, F-92200 Neuilly Sur Seine (FR); MACRON, Jonathan, F-75015 Paris (FR); WEBER, Claude, F-91490 Milly La Forêt (FR)
(74) Mandataire: Pittis, Olivier
(86) Numéro de dépôt international: PCT/FR2011/052934
(87) Numéro de publication internationale: WO 2012/098303

(56) Documents cités:
- WO-A1-2006/102707
- WO-A1-2010/096467
- WO-A1-2010/141983
- GB-A- 2 338 420
- US-A1- 2005 109 122
- US-A1- 2006 037 613

## Description

La présente invention concerne un procédé de surveillance d'un passage de gaz d'une installation de traitement de l'apnée obstructive du sommeil. Elle concerne également un appareil de suivi de l'observance dudit traitement.

Le syndrome d'apnée obstructive du sommeil (SAOS) est un trouble répandu affectant des millions d'adultes et d'enfants, qui se caractérise par une obstruction des voies aériennes supérieures, laquelle peut provoquer des ronflements ou des arrêts de la respiration durant le sommeil.

L'obstruction qui a lieu pendant le sommeil a deux causes principales qui sont le manque de tonus musculaire et la pesanteur. En effet, la présence excessive de tissus dans les voies aériennes supérieures et des déformations anatomiques aggravent les conséquences de ces facteurs. Ainsi, pendant le sommeil, particulièrement pendant le sommeil paradoxal, le corps se détend et les tissus musculaires, comme la langue et le voile du palais par exemple, perdent de leur rigidité. Par ailleurs, lorsque l'on dort en position allongée, l'effet de la pesanteur pousse ces tissus vers le fond de la gorge, ce qui ferme les voies aériennes supérieures.

Lorsqu'ils bloquent complètement les voies aériennes supérieures, ces tissus empêchent la personne de respirer au risque de provoquer une asphyxie. Mais, en général, la personne se réveille assez pour reprendre le contrôle de ses voies aériennes supérieures et respirer, avant de se rendormir. Chez une personne souffrant du SAOS, ce phénomène peut se produire des dizaines voire des centaines de fois par nuit mais habituellement la personne ne s'en rappelle pas au réveil.

Or, chaque obstruction prive le corps d'oxygène et le force donc à garder du dioxyde de carbone (CO₂) qu'il expulserait normalement lors des phases d'expiration. Il s'ensuit que l'équilibre gazeux du sang est perturbé et le corps est exposé à un environnement "toxique". Lorsque le corps "signale" qu'il a besoin de plus d'oxygène, le cerveau réveille le dormeur, la respiration reprend et la personne se rendort jusqu'à la prochaine obstruction. Ces obstructions entraînent également une augmentation du rythme cardiaque et de la pression artérielle, et éventuellement affaiblissent la capacité de réaction "automatique" du corps, ce qui se traduit par des apnées et hypopnées de plus en plus sévères.

Les micro-réveils cycliques que connaissent les personnes atteintes du SAOS affectent la qualité de leur sommeil. Les symptômes de la privation de sommeil chez les personnes atteintes du SAOS sont notamment une somnolence diurne excessive, un manque de concentration, une mauvaise mémoire, voire même un état dépressif. L'hypertension et la baisse du taux d'oxygène sanguin sont des symptômes fréquents pour les personnes qui souffrent d'apnée du sommeil, mais ce sont des symptômes difficiles à détecter. Il existe par ailleurs d'autres symptômes plus faciles à identifier tels que somnolence diurne, ronflements, apnées ou respiration irrégulière pendant le sommeil, perte de concentration.

Un traitement efficace du SAOS consiste à appliquer une pression positive d'air aux voies respiratoires du patient. La pression d'air agit comme un "coussin d'air" qui maintient ouvertes les voies respiratoires supérieures et empêche les apnées. Pour ce faire, on utilise typiquement un appareil de type à PPC (« Pression Positive Continue ») ou CPAP (en anglais pour « Continuous Positive Airway Pressure ») qui délivre de l'air légèrement pressurisé aux voies aériennes du patient, via une conduite souple, appelée circuit patient, reliée à un masque respiratoire nasal ou facial.

Cependant, l'air pressurisé délivré par de tels appareils peut être trop sec. Pour pallier cet inconvénient, les appareils de traitement utilisés comprennent souvent un humidificateur. Cet humidificateur humidifie l'air à pression positive continue dans l'appareil, réduisant l'irritation des voies nasales provoquée par le flux d'air à pression positive continue. En effet, dans certains cas, l'irritation assèche les voies nasales et peut provoquer un saignement. Elle peut également engendrer un oedème, une sécrétion excessive de mucus, une congestion ou des éternuements. L'irritation représente également un terrain propice au développement d'infections. Elle peut être cumulative, se renforçant au cours du temps.

La condensation ou l'accumulation d'eau dans le circuit patient, notamment au niveau d'un point bas de celui-ci, est courante lors de l'utilisation d'un tel humidificateur. La chaleur émise par l'humidificateur transporte l'eau en la transformant en vapeur d'eau. Si la pièce dans laquelle le patient dort est froide, la vapeur d'eau peut se liquéfier et s'accumuler dans le circuit patient entraînant une gêne pour le patient. En particulier, il a été déterminé que, si la présence d'eau condensée sur les parois du circuit peut provoquer une gêne limitée, l'obstruction du circuit provoque quant à elle une gêne plus sévère dont une conséquence est que le patient n'est pas correctement soigné.

US 2006/037613, ainsi que WO 2006/102707, divulguent un procédé de surveillance d'un passage de gaz d'une installation de traitement de l'apnée obstructive du sommeil selon le préambule de la revendication 1.

La présente invention vise à améliorer la situation en proposant un procédé et un dispositif permettant de détecter toute condensation ou accumulation intempestive d'eau dans le passage de gaz d'une installation de traitement de l'apnée obstructive du sommeil.

A cet effet, la présente invention a d'abord pour objet un procédé de surveillance d'un passage de gaz d'une installation de traitement de l'apnée obstructive du sommeil, comprenant les étapes de :
- mesure d'au moins un paramètre représentatif d'une obstruction du passage de gaz par de l'eau condensée; et
- détermination de la présence d'une obstruction à partir d'un traitement dudit au moins un paramètre ;
   caractérisé en ce qu'on mesure périodiquement la pression et/ou le débit du gaz dans au moins une partie du passage afin d'obtenir au moins un signal de pression et/ou de débit révélant une circulation de bulles d'air dans l'eau condensée, en tant que paramètre représentatif d'une obstruction du passage de gaz.

Ainsi, on peut utiliser comme paramètre représentatif d'une obstruction du passage de gaz :
- soit la pression du gaz dans au moins une partie du passage afin d'obtenir un signal de pression révélant la circulation de bulles d'air dans l'eau condensée. En effet, en raison de la pression assurée par l'appareil PPC, l'air traverse la zone du passage obstruée par l'eau condensée sous la forme de bulles, créant une perturbation du signal de pression dans le conduit.
- soit le débit. En effet, travailler sur le débit est équivalent à travailler sur la pression car les bulles d'air perturbent l'écoulement de l'air et donc le débit.

Dans la description qui suit, on illustre un mode de réalisation préféré de l'invention dans lequel le paramètre représentatif de l'obstruction du passage de gaz est la pression ; toutefois, on gardera à l'esprit que selon un autre mode de réalisation, le débit peut lui aussi être utilisé de façon similaire à la pression.

Avantageusement, on mesure périodiquement la pression du gaz. On peut utiliser, par exemple, une fréquence de mesure inférieure à 50 Hz, notamment de l'ordre de 25 Hz.

Ensuite, selon le mode de réalisation considéré :
- on filtre au moins un signal de pression et/ou de débit à l'aide d'un filtre passe-bande afin d'obtenir au moins un signal de pression ou de débit filtré;
- on évalue l'écart type dudit au moins signal de pression ou de débit filtré ;
- on compare l'écart type à au moins un seuil prédéterminé ; et
- on détermine la présence d'une obstruction si ledit écart type est supérieur à au moins un seuil prédéterminé.

L'invention a également pour objet un dispositif de surveillance d'un passage de gaz d'une installation de traitement de l'apnée obstructive du sommeil, comprenant :
- des moyens de mesure d'au moins un paramètre représentatif d'une obstruction du passage de gaz par de l'eau condensée; et
- des moyens de détermination de la présence d'une obstruction à partir d'un traitement dudit au moins un paramètre,
caractérisé en ce que les moyens de mesure d'au moins un paramètre représentatif d'une obstruction du passage de gaz comprennent :
- des moyens de mesure périodique de la pression du gaz dans au moins une partie du passage afin d'obtenir au moins un signal de pression révélant la circulation de bulles d'air dans l'eau condensée, et/ou
- des moyens de mesure périodique du débit du gaz dans au moins une partie du passage afin d'obtenir au moins un signal de débit révélant la circulation de bulles d'air dans l'eau condensée.

Selon un mode préféré de l'invention, les moyens de mesure d'un paramètre représentatif d'une obstruction du passage de gaz comprennent des moyens de mesure périodique de la pression du gaz dans au moins une partie du passage afin d'obtenir au moins un signal de pression révélant la circulation de bulles d'air dans l'eau condensée.

De préférence, les moyens de mesure de la pression comprennent un premier capteur de pression apte à mesurer périodiquement la pression du gaz au niveau de l'entrée du passage afin d'obtenir un premier signal de pression ; et/ou un deuxième capteur de pression apte à mesurer périodiquement la pression du gaz au niveau de la sortie du passage afin d'obtenir un deuxième signal de pression.

La mesure périodique se fait à une fréquence inférieure à 50 Hz, par exemple à une fréquence de 25 Hz.

De façon alternative, les moyens de mesure de débit comprennent un premier capteur de débit apte à mesurer périodiquement le débit du gaz à n'importe quel niveau ou endroit du passage.

Avantageusement, les moyens de détermination d'une obstruction comprennent :
- un filtre passe-bande pour filtrer le premier signal de pression et/ou le deuxième signal de pression et/ou le signal de débit afin d'obtenir respectivement au moins un premier signal de pression filtré et/ou au moins un deuxième signal de pression filtré et/ou au moins un signal de débit filtré;
- des moyens d'évaluation du ou des écarts types du premier signal et/ou du deuxième signal de pression filtrés et/ou un signal de débit filtré;
- des moyens de comparaison du ou desdits écarts types à au moins un seuil prédéterminé ; et
- des moyens de détermination de la présence d'une obstruction si l'écart type du premier signal de pression filtré et/ou l'écart type du deuxième signal de pression filtré et/ou l'écart type du signal de débit filtré est supérieur audit au moins un seuil prédéterminé.

De préférence, le filtre passe-bande a une largeur de bande comprise entre 2 et 9 Hz et de préférence entre 4 et 7 Hz, de préférence encore entre 6 et 7 Hz. Grâce à ce filtre passe-bande, les perturbations dues à la présence d'eau condensée, qui sont caractérisées par la présence de bulles d'air dans l'eau et qui présentent la fréquence filtrée, se distinguent de la respiration du patient ayant une fréquence basse inférieure à 1 Hz, puisqu'elle est généralement comprise entre 0,1 Hz et 0,8 Hz et des ronflements du patient ayant une fréquence élevée supérieure à 10 Hz, généralement de l'ordre de 100 Hz.

Il est à noter que les étapes et les éléments de traitement du signal sont les mêmes ou quasiment les mêmes lorsque l'on utilise un capteur de débit plutôt qu'un capteur de pression, notamment filtre passe-bande. La description précédente s'applique dès lors stricto sensu aussi au cas où on travaille sur le débit plutôt que sur la pression.

L'invention a par ailleurs aussi pour objet un appareil de suivi de l'observance d'un traitement de l'apnée obstructive du sommeil comprenant un boîtier, ledit boîtier comprenant :
- un passage de gaz comprenant une entrée apte à être raccordée à un appareil de traitement de l'apnée obstructive du sommeil et une sortie apte à être raccordée à un conduit vers un masque respiratoire ; et
- le dispositif de surveillance d'un passage de gaz de l'invention.

Connaître l'observance des patients, c'est-à-dire mesurer le temps réel pendant lequel ils suivent leur traitement est essentiel car les effets du traitement sont négligeables, voire nuls, si le patient n'observe pas son traitement pendant au moins quatre heures par nuit.

L'intégration d'un dispositif de surveillance tel que décrit plus haut dans un appareil de suivi de l'observance permet de reconnaître les périodes pendant lesquelles le patient n'est pas correctement soigné et les mesures de l'appareil sont perturbées en raison d'une obstruction du circuit.

Ainsi, les données d'observance issues de l'appareil peuvent être analysées de manière plus fiable et efficace.

Avantageusement, le boîtier comprend des moyens de détermination d'au moins une donnée de durée de traitement et d'au moins une donnée d'efficacité de traitement de l'apnée obstructive du sommeil.

Dans le cadre de l'invention :
- l'observance est la durée du traitement, par exemple le nombre d'heures ou de minutes par nuit, le nombre de jours par semaine ou par mois, le nombre d'heures ou de minutes moyen par nuit ou par jour, le nombre de jours pour lesquels la durée du traitement a été supérieure à quatre heures, le nombre d'heures de fonctionnement par jour de l'appareil de traitement ; et
- l'efficacité du traitement est une indication du nombre résiduel d'apnées obstructives et centrales, hypopnées, limitations de débit, ronflements et/ou de la pression moyenne de gaz appliquée, ainsi que des fuites de gaz éventuelles s'étant produits durant la période de traitement considérée.

De préférence, le boîtier comprend en outre :
- des moyens de stockage de la donnée de durée de traitement journalier, de la donnée d'efficacité de traitement de l'apnée obstructive du sommeil et d'une donnée relative à la présence d'une dite obstruction dans le passage ; et
- des moyens de transmission desdites données vers un serveur distant.

Ce serveur distant est notamment situé dans le centre de soins dans lequel le patient est suivi ou chez un prestataire de services permettant un accès à distance du médecin traitant au serveur. Le médecin traitant dispose ainsi en temps réel des données d'observance du traitement par le patient et sait si une obstruction par de l'eau condensée présente dans le circuit patient gêne son traitement.

Selon le cas, l'appareil de suivi d'observance peut comprendre l'une ou plusieurs des caractéristiques suivantes :
- les moyens de stockage de données comprennent au moins une puce mémoire ou une carte mémoire, de préférence enfichable, par exemple une carte SD ou analogue ;
- les moyens de transmission comprennent un système émetteur sans fil, notamment de type radiofréquence, bluetooth, Zigbee, wifi, GSM ou GPRS, et une antenne permettant d'assurer une transmission sans fil des données adaptée au type d'émetteur et insérée dans le boîtier ;
- le système émetteur radiofréquence comprend un modem GSM ou GPRS intégré ou extérieur au boîtier ; et
- il comprend en outre un ou des indicateurs, telles des LEDs de couleurs, par exemple rouge et verte, fournissant à l'utilisateur une information relative à l'efficacité du traitement.

Enfin, l'invention porte aussi sur une installation de traitement de l'apnée obstructive du sommeil comprenant un appareil selon l'invention, raccordé, via le boitier, à un appareil de traitement de l'apnée obstructive du sommeil et par ailleurs à un conduit, de référence flexible, de gaz relié à un masque respiratoire.

De préférence, l'appareil de traitement de l'apnée obstructive du sommeil est une CPAP.

Avantageusement, l'installation comporte un humidificateur de gaz.

L'invention va maintenant être décrite de manière plus détaillée en référence aux Figures annexées parmi lesquelles :
- la figure 1 est un schéma synoptique illustrant la structure d'un appareil de suivi de l'observance selon un mode de réalisation de l'invention ;
- la figure 2 est un schéma synoptique illustrant la mise en oeuvre de l'appareil de suivi de l'observance de la figure 1; et
- la figure 3 est un organigramme illustrant le fonctionnement du procédé de détection selon un mode de réalisation de l'invention.

Selon un mode de réalisation, un appareil de suivi 2 de l'observance d'un traitement de l'apnée obstructive du sommeil (AOS) selon la présente invention est formé d'un boîtier 4 venant se raccorder sur le trajet du gaz respiratoire, typiquement de l'air sous pression, c'est-à-dire sur le circuit patient reliant un appareil de traitement 6 de l'AOS au masque respiratoire 8, en général nasal, équipant un patient à traiter.

L'appareil de traitement 6 est muni d'un humidificateur 9 qui humidifie l'air pour éviter une irritation des voies nasales du patient.

La connexion de l'appareil de suivi 2 au circuit patient de l'appareil de traitement 6 est réalisée de préférence au moyen de tubes flexibles à embouts classiques, par exemple des embouts de diamètre égal à 22 mm et conformes à la norme ISO 5356-1.

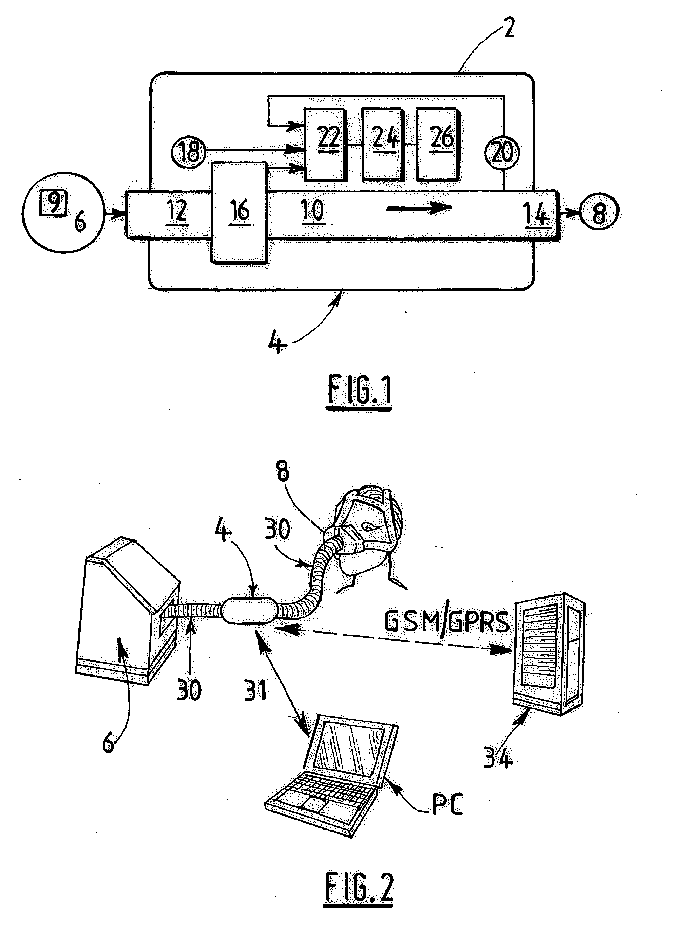

Comme illustré sur la figure 1, le boîtier 4 comprend un passage 10 interne de gaz avec une entrée 12 et une sortie 14 au sein duquel transite le gaz débité par l'appareil 6 de traitement de l'AOS, avant d'être envoyé au patient.

Un dispositif de détermination de débit 16, couramment appelé capteur de débit, pourra être agencé dans le boîtier 4 et relié au passage 10 de manière à permettre une mesure du débit du gaz circulant à l'intérieur dudit passage 10, c'est-à-dire entre l'entrée 12 et la sortie 14 du passage 10.

Par ailleurs, un premier capteur de pression absolue 18 et un deuxième capteur de pression absolue 20 sont également agencés sur ce passage 10 pour mesurer périodiquement la pression du gaz respectivement au niveau de l'entrée 12 et de la sortie 14 du passage 10 fournissant ainsi un premier signal et un deuxième signal de pression, respectivement.

Le capteur de débit 16 et les capteurs de pression 18, 20 sont raccordés par ailleurs à des moyens de traitement 22, tels qu'un microcontrôleur, par exemple le microcontrôleur MSP430 de Texas Instruments, mettant en oeuvre des algorithmes capables de traiter les mesures de pression et de débit afin d'en déduire, entre autres, la durée de traitement journalier et l'efficacité du traitement de l'AOS du patient.

Selon l'invention, les moyens de traitement 22 sont également aptes à traiter le ou les signaux de pressions issus des capteurs de pression 18, 20 ou le ou les signaux de débit du capteur de débit 16 pour détecter la présence d'eau condensée dans le passage 10, notamment la présence de bulles d'air dans l'eau.

Le capteur de débit 16 peut être un débitmètre à fil chaud ou un débitmètre déprimogène.

Par ailleurs, les capteurs de pression 18,20 peuvent être par exemple des capteurs BMP085 commercialisés par la société Bosch.

Des moyens de stockage de données 24 sont utilisés pour mémoriser tout ou partie des données ainsi mesurées, par exemple une puce mémoire d'enregistrement de données ou une carte mémoire enfichable, notamment une carte mémoire flash de 8 Go de SST.

Par ailleurs, des moyens de transmission 26, par exemple un émetteur radiofréquence et son antenne, sont prévus pour transmettre, de préférence via une transmission sans fil, tout ou partie desdites données vers un récepteur situé à distance, tel un ordinateur ou un serveur, comme illustré sur la figure 2. L'émetteur radiofréquence peut, par exemple, être équipé d'une antenne de 870 MHz de PHYCOMP.

Des moyens d'alimentation en courant électrique, non représentés, sont raccordés électriquement aux capteurs 16, 18, 20, aux moyens de stockage de données 24 et aux moyens de transmission 26 pour assurer l'alimentation électrique de l'appareil de suivi 2, par exemple une alimentation électrique à basse tension comprenant une ou plusieurs batteries, piles...

De préférence, l'appareil de suivi 2 a pour fonction de mesurer, indépendamment de l'appareil de traitement 6 de l'AOS, c'est-à-dire sans utiliser d'informations ou de données internes à cet appareil de traitement 6, et de communiquer à distance les informations relatives à l'observance et à l'efficacité du traitement, c'est-à-dire la durée de traitement effective du patient, ainsi que les événements comme les apnées, hypopnées, limitations de débit, ronflements, fuites, etc.

De manière remarquable, l'appareil de suivi de l'observance 2 détermine et communique également des informations relatives à la présence d'eau sous forme liquide dans le passage de gaz 10, plus particulièrement la présence d'eau liquide obstruant le circuit patient à travers laquelle l'air généré par l'appareil de traitement 6 passe en faisant des bulles.

Cela permet d'obtenir un suivi quotidien et une sécurité dans le suivi du traitement du patient à domicile grâce à la possibilité d'alarmer le patient et son centre de soins ou le prestataire de services en cas de non suivi de la prescription.

Ainsi que cela apparaît sur la figure 2, le boîtier 4 de l'appareil de suivi de l'observance 2 selon la présente invention vient s'incorporer sur le trajet du gaz, c'est-à-dire sur la ou les conduites 30 flexibles d'acheminement de gaz, entre l'appareil de traitement 6 distribuant de l'air, notamment sous pression positive continue, et le patient équipé du masque nasal ou facial 8, et permet de mesurer et enregistrer le temps de traitement journalier, ainsi que l'efficacité du traitement.

L'appareil de suivi 2 est conçu pour pouvoir s'adapter à tout type d'appareil de traitement 6 du SAOS, c'est-à-dire les ventilateurs de types CPAP, BiPAP et analogues.

L'appareil de suivi 2 possède une capacité d'enregistrement de plusieurs mois, de préférence d'au moins 1 an, laquelle peut être encore étendue.

Il peut communiquer les données enregistrées par une liaison de transmission d'information 31, par exemple radiofréquence RF, à une fréquence d'émission de 868 MHz ou 2.4 GHz, ou de préférence une liaison USB, vers un ordinateur, un PDA, un serveur ou tout autre moyen capable d'enregistrer directement les données transmises, comme montré sur la figure 2. En fait, l'appareil de suivi 2 transmet à distance, à l'aide d'un modem GSM ou GPRS intégré, les enregistrements de données d'observance et d'efficacité du traitement du patient jusqu'au centre de soins ou au prestataire de services par exemple, où un serveur 34 adapté permet de générer des rapports d'observance et d'efficacité de traitement.

Un patient est considéré comme étant bien en cours de traitement si deux conditions sont remplies, à savoir :
- si l'on détecte la respiration du patient par un algorithme spécifique de traitement des signaux de débit et de pression mesurés dans le circuit patient permettant de déduire de ces signaux que le patient porte bien le masque nasal ou naso-buccal ; et
- si l'on détecte une pression minimale de traitement correspondant à un appareil de traitement qui est en marche et qui fonctionne correctement.

Ces deux conditions peuvent être déterminées à l'aide de mesures de pression et de débit dans le circuit patient grâce à l'appareil de suivi 2. Un tel agencement permet de déduire les variations de pression et de débit liées, d'une part, à la délivrance d'air et, d'autre part, aux inspirations et expirations du patient dans le circuit patient.

L'efficacité du traitement est alors déduite des variations de pression et de débit liées aux inspirations/expirations du patient et du niveau de pression de traitement. Elle est mesurée par la détection du nombre d'apnées, d'hypopnées, de limitations de débit, de fuites du circuit patient ou du masque, et du temps de ronflement survenant pendant le traitement.

Cependant, le traitement lui-même ainsi que la mesure de son efficacité peuvent être perturbés par l'accumulation d'eau liquide issue de la condensation de la vapeur d'eau dans le passage 10, généralement au niveau d'un coude situé en partie basse du circuit, entraînant la circulation de bulles d'air dans l'eau et l'obstruction du passage 10.

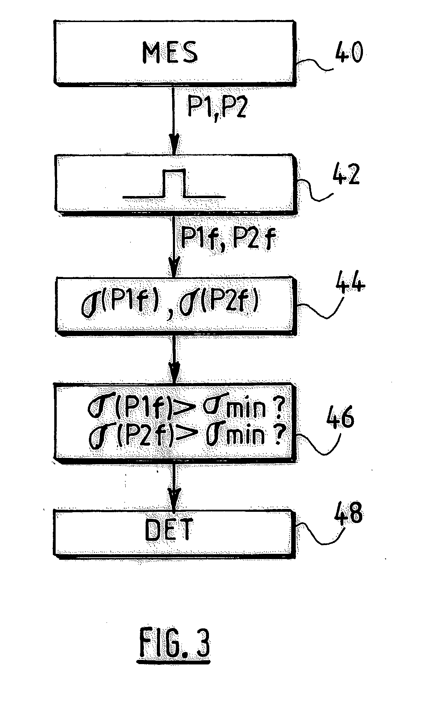

Le procédé de l'invention, décrit en référence à l'organigramme de la figure 3, permet de détecter, à partir du débit et/ou de la pression mesurés, la présence de cette eau de condensation dans le passage 10.

Ce procédé est avantageusement mis en oeuvre dans le boîtier 4 de l'appareil de suivi 2 de l'observance.

Ainsi, l'utilisation du ou des signaux de pression émanant du ou des capteurs de pression 18, 20 pour détecter la présence d'eau de condensation, peut se faire de la façon suivante.

Lors d'une étape 40, le premier et deuxième capteurs de pression 18, 20 mesurent périodiquement, notamment à une fréquence inférieure à 50 Hz, par exemple à une fréquence de 25 Hz, la pression de l'air respectivement au niveau de l'entrée 12 et de la sortie 14 du passage 10. Deux signaux de pression P1 et P2 sont ainsi obtenus, le signal P1 représentant la pression à l'entrée 12 et le signal P2 représentant la pression à la sortie 14.

Lors d'une étape 42, les moyens de traitement 22 filtrent les signaux de pression P1, P2 à l'aide d'un filtre passe-bande ayant une largeur de bande comprise entre 2 et 9Hz et de préférence entre 4 et 7 Hz, voire 6 à 7 Hz, pour obtenir des signaux de pression P1_{f}, P2_{f} filtrés, respectivement.

Grâce à ce filtrage passe-bande à l'étape 42, les perturbations générées par la respiration (situées à des fréquences basses inférieures 1 Hz) du patient et par ses ronflements (situées à des fréquences hautes supérieures 10 Hz) sont éliminées. Ainsi, les seules perturbations présentes dans les signaux de pression filtrés correspondent à la présence de bulles d'air dans de l'eau condensée.

A l'étape 44, les moyens de traitement 22 calculent l'écart type du premier signal de pression filtré P1_{f} et l'écart type du deuxième signal de pression filtré P2_{f}.

A l'étape 46, les moyens de traitement 22 comparent les écarts types des deux signaux de pression filtrés à un seuil prédéterminé σₘᵢₙ, typiquement compris entre 5 et 7.

Si l'un au moins des deux écarts types est supérieur au seuil, alors les moyens de traitement 22 déterminent, à l'étape 48, que des bulles d'air dans de l'eau condensée sont présentes dans le passage 10, ce qui indique la présence d'un « bouchon » d'eau condensée.

Les moyens de traitement 22 stockent dans les moyens de stockage 24 des données relatives à cette présence d'eau, notamment la durée de présence.

Les moyens de transmission 24 transmettent ces données vers le serveur distant 34 afin que le médecin traitant sache que de l'eau condensée dans le circuit patient gêne le traitement.

La présente invention concerne donc un procédé de surveillance d'un passage de gaz d'une installation de traitement de l'apnée obstructive du sommeil (AOS) en vue d'y détecter une éventuelle présence d'eau de condensation, ainsi qu'un appareil de suivi de l'observance dudit traitement mettant en oeuvre ce procédé.

## Revendications

1. Procédé de surveillance d'un passage de gaz (10) d'une installation de traitement (6) de l'apnée obstructive du sommeil, comprenant les étapes de :
- mesure (40) d'au moins un paramètre représentatif d'une obstruction du passage de gaz (10) par de l'eau condensée; et
- détermination (48) de la présence d'une obstruction à partir d'un traitement dudit au moins un paramètre,
**caractérisé en ce qu'**on mesure (40) périodiquement la pression et/ou le débit (Q) du gaz dans au moins une partie du passage (10) afin d'obtenir un signal de pression (P1,P2) et/ou de débit (Q) révélant la circulation de bulles d'air dans l'eau condensée, en tant que paramètre représentatif d'une obstruction du passage de gaz (10).

2. Procédé selon la revendication 1, **caractérisé en ce qu'**on mesure (40) périodiquement la pression du gaz dans au moins une partie du passage (10) en tant que paramètre représentatif d'une obstruction du passage de gaz (10).

3. Procédé selon l'une des revendications 1 ou 2, **caractérisé en ce que** :
- on filtre (42) au moins un signal de pression (P1, P2) et/ou au moins un signal de débit (Q) mesuré à l'aide d'un filtre passe-bande afin d'obtenir au moins un signal de pression (P1_{f}, P2_{f}) ou de débit (Q_{f}) filtré ;
- on évalue (44) l'écart type dudit au moins un signal de pression (P1_{f}, P2_{f}) ou de débit (Q_{f}) filtré ;
- on compare (46) ledit écart type à au moins un seuil prédéterminé (σₘᵢₙ); et
- on détermine (48) la présence d'une obstruction si ledit écart type est supérieur audit au moins un seuil prédéterminé.

4. Dispositif de surveillance d'un passage de gaz (10) issu d'une installation de traitement (6) de l'apnée obstructive du sommeil, comprenant :
- des moyens de mesure (18, 20) d'au moins un paramètre représentatif d'une obstruction du passage de gaz (10) par de l'eau condensée; et
- des moyens de détermination (22) de la présence d'une obstruction à partir d'un traitement dudit au moins un paramètre,
**caractérisé en ce que** les moyens de mesure d'au moins un paramètre représentatif d'une obstruction du passage de gaz (10) comprennent :
- des moyens de mesure périodique (18,20) de la pression du gaz dans au moins une partie du passage (10) afin d'obtenir au moins un signal de pression (P1,P2) révélant la circulation de bulles d'air dans l'eau condensée, et/ou
- des moyens de mesure périodique (16) du débit du gaz dans au moins une partie du passage (10) afin d'obtenir au moins un signal de débit (Q) révélant la circulation de bulles d'air dans l'eau condensée.

5. Dispositif selon la revendication 4, **caractérisé en ce que** les moyens de mesure de la pression comprennent :
- un premier capteur de pression (18) apte à mesurer périodiquement la pression du gaz au niveau de l'entrée (12) du passage afin d'obtenir au moins un premier signal de pression (P1); et/ou
- un deuxième capteur de pression (20) apte à mesurer périodiquement la pression du gaz au niveau de la sortie (14) du passage afin d'obtenir au moins un deuxième signal de pression (P2).

6. Dispositif selon la revendication 4, **caractérisé en ce que** les moyens de mesure (16) du débit du gaz comprennent un capteur de débit.

7. Dispositif selon l'une des revendications 4 à 6, **caractérisé en ce que** les moyens de détermination de la présence d'une obstruction comprennent :
- un filtre passe-bande pour filtrer au moins le premier signal de pression (P1) et/ou le deuxième signal de pression (P2), et/ou le signal de débit (Q) afin d'obtenir respectivement au moins un premier signal de pression filtré (P1_{f}) et/ou au moins un deuxième signal de pression filtré (P2_{f}) et/ou au moins un signal de débit filtré (Q);
- des moyens d'évaluation du ou des écarts types du premier signal (P1_{f}), du deuxième signal (P2_{f}) de pression filtrés et/ou du signal (Q_{f}) de débit filtré;
- des moyens de comparaison dudit ou desdits écarts types à au moins un seuil prédéterminé (σₘᵢₙ) ; et
- des moyens de détermination de la présence d'une obstruction si l'écart type du premier signal de pression filtré (P1_{f}) et/ou l'écart type du deuxième signal de pression filtré (P2_{f}) et/ou l'écart type du signal de débit filtré (Q_{f}) est supérieur audit au moins un seuil prédéterminé (σₘᵢₙ).

8. Dispositif selon l'une des revendications 4 à 7, **caractérisé en ce que** le filtre passe-bande a une largeur de bande comprise entre 2 et 9 Hz et de préférence entre 4 et 7 Hz.

9. Appareil de suivi (2) de l'observance d'un traitement de l'apnée obstructive du sommeil comprenant un boîtier (4), ledit boîtier (4) comportant un passage de gaz (10) comprenant une entrée (12) apte à être raccordée à un appareil de traitement (6) de l'apnée obstructive du sommeil et une sortie (14) apte à être raccordée à un conduit (30) vers un masque respiratoire (8); et un dispositif de surveillance selon l'une quelconque des revendications 4 à 8.

10. Appareil selon la revendication 9, **caractérisé en ce que** le boîtier (4) comprend des moyens de détermination (22) d'au moins une donnée de durée de traitement et d'au moins une donnée d'efficacité de traitement de l'apnée obstructive du sommeil.

11. Appareil selon l'une des revendications 9 ou 10, **caractérisé en ce que** le boîtier (4) comprend des moyens de stockage (24) d'au moins une donnée de durée de traitement journalier, d'au moins une donnée d'efficacité de traitement de l'apnée obstructive du sommeil et d'au moins une donnée relative à présence d'une obstruction dans le passage (10).

12. Appareil selon l'une des revendications 9 à 11, **caractérisé en ce que** le boîtier (4) comprend des moyens de transmission (26) d'au moins une desdites données vers un serveur distant (34).

13. Installation de traitement (6) de l'apnée obstructive du sommeil comprenant un appareil selon l'une des revendications 9 à 12, raccordé à un appareil de traitement (6) de l'apnée obstructive du sommeil et par ailleurs à un conduit (30) de gaz relié à un masque respiratoire (8).

14. Installation selon la revendication 13, **caractérisée en ce que** l'appareil de traitement (6) de l'apnée obstructive du sommeil est une CPAP.

15. Installation selon l'une des revendications 13 ou 14, **caractérisé en ce qu'**elle comporte un humidificateur (9).

## Patentansprüche

1. Verfahren zum Überwachen eines Gasdurchlasses (10) aus einer Anlage zum Behandeln (6) der obstruktiven Schlafapnoe, umfassend die folgenden Schritte:
- Messen (40) mindestens eines Parameters, der für eine Obstruktion des Gasdurchlasses (10) durch Kondenswasser repräsentativ ist; und
- Bestimmen (48) des Vorliegens einer Obstruktion ausgehend von einer Verarbeitung des mindestens einen Parameters,
**gekennzeichnet durch** das periodische Messen (40) des Drucks und/oder der Durchflussmenge (Q) des Gases in mindestens einem Teil des Durchlasses (10), um ein Drucksignal (P1, P2) und/oder ein Durchflussmengensignal (Q) zu erhalten, das die Zirkulation von Luftblasen in dem Kondenswasser als Parameter, der für eine Obstruktion des Gasdurchlasses (10) repräsentativ ist, anzeigt.

2. Verfahren nach Anspruch 1, **gekennzeichnet durch** das periodische Messen (40) des Drucks des Gases in mindestens einem Teil des Durchlasses (10) als Parameter, der für eine Obstruktion des Gasdurchlasses (10) repräsentativ ist.

3. Verfahren nach einem der Ansprüche 1 oder 2, **gekennzeichnet durch**:
- Filtern (42) mindestens eines gemessenen Drucksignals (P1, P2) und/oder mindestens eines gemessenen Durchflussmengensignals (Q) mit Hilfe eines Bandpassfilters, um mindestens ein gefiltertes Drucksignal (P1_{f}, P2_{f}) oder ein gefiltertes Durchflussmengensignal (Q_{f}) zu erhalten;
- Auswerten (44) der Standardabweichung des mindestens einen gefilterten Drucksignals (P1_{f}, P2_{f}) oder gefilterten Durchflussmengensignals (Q_{f});
- Vergleichen (46) der Standardabweichung mit mindestens einem vorbestimmten Schwellenwert (σₘᵢₙ); und
- Bestimmen (48) des Vorliegens einer Obstruktion, wenn die Standardabweichung größer als mindestens ein vorbestimmter Schwellenwert ist.

4. Vorrichtung zum Überwachen eines Gasdurchlasses (10) aus einer Anlage zum Behandeln (6) der obstruktiven Schlafapnoe, umfassend:
- Mittel zum Messen (18, 20) mindestens eines Parameters, der für eine Obstruktion des Gasdurchlasses (10) durch Kondenswasser repräsentativ ist; und
- Mittel zum Bestimmen (22) des Vorliegens einer Obstruktion ausgehend von der Verarbeitung des mindestens einen Parameters,
**dadurch gekennzeichnet, dass** die Mittel zum Messen mindestens eines Parameters, der für eine Obstruktion des Gasdurchlasses (10) durch Kondenswasser repräsentativ ist, Folgendes umfassen:
- Mittel zum periodischen Messen (18, 20) des Drucks des Gases in mindestens einem Teil des Durchlasses (10), um mindestens ein Drucksignal (P1, P2) zu erhalten, das die Zirkulation von Luftblasen in dem Kondenswasser anzeigt, und/oder
- Mittel zum periodischen Messen (16) der Durchflussmenge des Gases in mindestens einem Teil des Durchlasses (10), um mindestens ein Durchflussmengensignal (Q) zu erhalten, das die Zirkulation von Luftblasen in dem Kondenswasser ergibt.

5. Vorrichtung nach Anspruch 4, **dadurch gekennzeichnet, dass** die Mittel zum Messen des Drucks Folgendes umfassen:
- einen ersten Drucksensor (18) zum periodischen Messen des Drucks des Gases im Bereich des Eingangs (12) des Durchlasses, um mindestens ein erstes Drucksignal (P1) zu erhalten; und/oder
- einen zweiten Drucksensor (20) zum periodischen Messen des Drucks des Gases im Bereich des Ausgangs (14) des Durchlasses, um mindestens ein zweites Drucksignal (P2) zu erhalten.

6. Vorrichtung nach Anspruch 4, **dadurch gekennzeichnet, dass** die Mittel zum Messen (16) der Durchflussmenge des Gases einen Gasdurchfluss-Sensor umfassen.

7. Vorrichtung nach einem der Ansprüche 4 bis 6, **dadurch gekennzeichnet, dass** die Mittel zum Bestimmen des Vorliegens einer Obstruktion Folgendes umfassen:
- einen Bandpassfilter zum Filtern mindestens des ersten Drucksignals (P1) und/oder des zweiten Drucksignals (P2) und/oder des Durchflussmengensignals (Q), um mindestens ein erstes gefiltertes Drucksignal (P1_{f}) bzw. mindestens ein zweites gefiltertes Drucksignal (P2_{f}) bzw. mindestens ein gefiltertes Durchflussmengensignal (Q) zu erhalten;
- Mittel zum Auswerten der Standardabweichung(en) des ersten gefilterten Drucksignals (P1_{f}), des zweiten gefilterten Drucksignals (P2_{f}) und/oder des gefilterten Durchflussmengensignals (Q_{f});
- Mittel zum Vergleichen der Standardabweichung(en) mit mindestens einem vorbestimmten Schwellenwert (σₘᵢₙ); und
- Mittel zum Bestimmen des Vorliegens einer Obstruktion, wenn die Standardabweichung des ersten gefilterten Drucksignals (P1_{f}) und/oder die Standardabweichung des zweiten gefilterten Drucksignals (P2_{f}) und/oder die Standardabweichung des gefilterten Durchflussmengensignals (Q_{f}) größer als der mindestens eine vorbestimmte Schwellenwert (σₘᵢₙ) ist.

8. Vorrichtung nach einem der Ansprüche 4 bis 7, **dadurch gekennzeichnet, dass** der Bandpassfilter eine Bandbreite hat, die zwischen 2 und 9 Hz und bevorzugt zwischen 4 und 7 Hz beträgt.

9. Gerät zum Verfolgen (2) der Einhaltung einer Behandlung der obstruktiven Schlafapnoe, umfassend ein Gehäuse (4), wobei das Gehäuse (4) einen Gasdurchlass (10) aufweist, der einen Eingang (12) für die Verbindung mit einem Gerät zum Behandeln (6) der obstruktiven Schlafapnoe und einen Ausgang (14) für die Verbindung mit einer Leitung (30) zu einer Beatmungsmaske (8) umfasst; und eine Überwachungsvorrichtung nach einem der Ansprüche 4 bis 8 aufweist.

10. Gerät nach Anspruch 9, **dadurch gekennzeichnet, dass** das Gehäuse (4) Mittel zum Bestimmen (22) mindestens eines Datenelements für die Behandlungsdauer und mindestens eines Datenelements für die Wirksamkeit der Behandlung der obstruktiven Schlafapnoe umfasst.

11. Gerät nach einem der Ansprüche 9 oder 10, **dadurch gekennzeichnet, dass** das Gehäuse (4) Mittel zum Speichern (24) mindestens eines Datenelements für die tägliche Behandlungsdauer, mindestens eines Datenelements für die Wirksamkeit der Behandlung der obstruktiven Schlafapnoe und mindestens eines Datenelements für das Vorliegen einer Obstruktion in dem Durchlass (10) umfasst.

12. Gerät nach einem der Ansprüche 9 bis 11, **dadurch gekennzeichnet, dass** das Gehäuse (4) Mittel zum Senden (26) mindestens eines der Datenelemente zu einem Fernserver (34) umfasst.

13. Anlage zum Behandeln (6) der obstruktiven Schlafapnoe, umfassend ein Gerät nach einem der Ansprüche 9 bis 12, das verbunden ist mit einem Gerät zum Behandeln (6) der obstruktiven Schlafapnoe und des Weiteren mit einer Gasleitung (30), die mit einer Beatmungsmaske (8) verbunden ist.

14. Anlage nach Anspruch 13, **dadurch gekennzeichnet, dass** das Gerät zum Behandeln (6) der obstruktiven Schlafapnoe ein CPAP-Gerät ist.

15. Anlage nach einem der Ansprüche 13 oder 14, **dadurch gekennzeichnet, dass** sie einen Luftbefeuchter (9) umfasst.

## Claims

1. Method for monitoring a gas passage (10) of an obstructive sleep apnoea treatment unit (6) comprising the steps of:
- measuring (40) at least one parameter representing an obstruction of the gas passage (10) by condensed water; and
- determining (48) the presence of an obstruction by processing said at least one parameter,
**characterised in that** the pressure and/or the flow (Q) of the gas in at least one part of the passage (10) is periodically measured (40) in order to obtain a pressure signal (P1, P2) and/or a flow signal (Q) indicating the circulation of air bubbles in the condensed water as a parameter representing an obstruction of the gas passage (10).

2. Method according to claim 1, **characterised in that** the pressure of the gas is periodically measured (40) in at least one part of the passage (10) as a parameter representing an obstruction of the gas passage (10).

3. Method according to either claim 1 or claim 2, **characterised in that**:
- at least one measured pressure signal (P1, P2) and/or at least one measured flow signal (Q) is filtered (42) by means of a band-pass filter in order to obtain at least one filtered pressure signal (P1_{f}, P2_{f}) or flow signal (Q_{f});
- the standard deviation of said at least one filtered pressure signal (P1_{f}, P2_{f}) or flow signal (Q_{f}) is evaluated (44);
- said standard deviation is compared (46) with at least one predetermined threshold (σₘᵢₙ); and
- the presence of an obstruction is determined (48) if said standard deviation is greater than said at least one predetermined threshold.

4. Device for monitoring a gas passage (10) from an obstructive sleep apnoea treatment unit (6) comprising:
- means (18, 20) for measuring at least one parameter representing an obstruction of the gas passage (10) by condensed water; and
- means (22) for determining the presence of an obstruction by processing said at least one parameter,
**characterised in that** the means for measuring at least one parameter representing an obstruction of the gas passage (10) comprise:
- means (18, 20) for periodically measuring the pressure of the gas in at least one part of the passage (10) in order to obtain at least one pressure signal (P1, P2) indicating the circulation of air bubbles in the condensed water, and/or
- means (16) for periodically measuring the gas flow in at least one part of the passage (10) in order to obtain at least one flow signal (Q) indicating the circulation of air bubbles in the condensed water.

5. Device according to claim 4, **characterised in that** the means for measuring the pressure comprise:
- a first pressure sensor (18) capable of periodically measuring the pressure of the gas in the region of the inlet (12) of the passage in order to obtain at least one first pressure signal (P1); and/or
- a second pressure sensor (20) capable of periodically measuring the pressure of the gas in the region of the outlet (14) of the passage in order to obtain at least one second pressure signal (P2).

6. Device according to claim 4, **characterised in that** the means (16) for measuring the gas flow comprise a flow sensor.

7. Device according to any of claims 4 to 6, **characterised in that** the means for determining the presence of an obstruction comprise:
- a band-pass filter for filtering at least the first pressure signal (P1) and/or the second pressure signal (P2), and/or the flow signal (Q) in order to obtain at least one first filtered pressure signal (P1_{f}) and/or at least one second filtered pressure signal (P2_{f}) and/or at least one filtered flow signal (Q) respectively;
- means for evaluating the standard deviation(s) of the first filtered pressure signal (P1_{f}), the second filtered pressure signal (P2_{f}) and/or the filtered flow signal (Q_{f});
- means for comparing said standard deviation(s) with at least one predetermined threshold (σₘᵢₙ); and
- means for determining the presence of an obstruction if the standard deviation of the first filtered pressure signal (P1_{f}) and/or the standard deviation of the second filtered pressure signal (P2_{f}) and/or the standard deviation of the filtered flow signal (Q_{f}) is greater than said at least one predetermined threshold (σₘᵢₙ).

8. Device according to any of claims 4 to 7, **characterised in that** the band-pass filter has a bandwidth of between 2 and 9 Hz, and preferably of between 4 and 7 Hz.

9. Apparatus (2) for tracking compliance with a treatment of obstructive sleep apnoea comprising a housing (4), said housing (4) having a gas passage (10) comprising an inlet (12) capable of being connected to an obstructive sleep apnoea treatment apparatus (6) and an outlet (14) capable of being connected to a conduit (30) leading to a respirator mask (8); and a monitoring device according to any of claims 4 to 8.

10. Apparatus according to claim 9, **characterised in that** the housing (4) comprises means (22) for determining at least one piece of data regarding the duration of the treatment and at least one piece of data regarding the effectiveness of the obstructive sleep apnoea treatment.

11. Apparatus according to either claim 9 or claim 10, **characterised in that** the housing (4) comprises means (24) for storing at least one piece of data regarding the daily treatment duration, at least one piece of data regarding the effectiveness of the obstructive sleep apnoea treatment and at least one piece of data relating to the presence of an obstruction in the passage (10).

12. Apparatus according to any of claims 9 to 11, **characterised in that** the housing (4) comprises means (26) for transmitting at least one of said pieces of data to a remote server (34).

13. Obstructive sleep apnoea treatment unit (6) comprising an apparatus according to any of claims 9 to 12, which is connected to an obstructive sleep apnoea treatment apparatus (6) and also to a gas conduit (30) which is connected to a respirator mask (8).

14. Unit according to claim 13, **characterised in that** the obstructive sleep apnoea treatment apparatus (6) is a CPAP.

15. Unit according to either claim 13 or claim 14, **characterised in that** it comprises a humidifier (9).
